# EUROPEAN PATENT APPLICATION

(11) **EP 4 215 611 A1**
(43) Date of publication of application: **26.07.2023**
(21) Application number: 22152247.7
(22) Date of filing: 19.01.2022
(51) Int. Cl.: C12N 15/10, C12N 9/22, C12N 15/63, C12N 15/80

(54) **MODIFICATION OF THE GENOME OF A FILAMENTOUS FUNGUS**

(71) Applicant: BRAIN Biotech AG, 64673 Zwingenberg (DE)
(72) Inventor: SCHOLZ, Paul, 64673 Zwingenberg (DE); ZUREK, Christian, 64673 Zwingenberg (DE); HEGER, Rebecca, Marie, 64673 Zwingenberg (DE)
(74) Representative: Weickmann & Weickmann PartmbB

(57) **Abstract**

The present invention relates to a method for modifying a nucleotide sequence at a target site in the genome of the cell of a filamentous fungus comprising introducing into said cell one or more nucleic molecules comprising a first and a second expression cassette, wherein the first expression cassette comprises a nucleic acid molecule encoding a class 2, type V RNA-guided DNA endonuclease; and wherein the second expression cassette comprises a nucleic acid molecule encoding a DNA-targeting and self-splicing RNA comprising from the 5'-end to the 3'-end (a) a first self-splicing sequence, (b) a stem loop sequence of SEQ ID NO: 7 or 8 or a sequence being at least 75% identical thereto, (c) a nucleotide sequence that is complementary to a sequence at the target site; and (d) a second self-splicing sequence.

## Description

The present invention relates to a method for modifying a nucleotide sequence at a target site in the genome of the cell of a filamentous fungus comprising introducing into said cell one or more nucleic molecules comprising a first and a second expression cassette, wherein the first expression cassette comprises a nucleic acid molecule encoding a class 2, type V RNA-guided DNA endonuclease; and wherein the second expression cassette comprises a nucleic acid molecule encoding a DNA-targeting and self-splicing RNA comprising from the 5'-end to the 3'-end (a) a first self-splicing sequence, (b) a stem loop sequence of SEQ ID NO: 7 or 8 or a sequence being at least 75% identical thereto, (c) a nucleotide sequence that is complementary to a sequence at the target site; and (d) a second self-splicing sequence.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Filamentous fungi have the ability to grow in many different environments, including biological wastes, soil, and plant and algal biomass. They also have the ability to grow on a wide variety of inexpensive substrates and produce a rich repertoire of interesting metabolites and enzymes, which has aroused considerable interest on the part of the scientific community for their exploitation as production organisms in biotechnology. Nowadays, filamentous fungi are utilized as cell factories for the production of many industrially relevant compounds, such as organic acids, proteins, enzymes, exopolysaccharides, and secondary metabolites and fungi such as *Aspergillus niger*, *Aspergillus oryzae*, and *Trichoderma* reesei have become indispensable for enzyme production at industrial level (Frisvad et al., 2018). Fungal enzymes currently make up more than half of the enzymes used in industrial applications, reflecting the importance of filamentous fungi as cell factories (Sandra Garrigues et al., Encyclopedia of Mycology, 2021, ISBN 978-0-323-85180-0).

Genetic engineering for strain improvement is a very powerful tool for increasing production levels, producing novel compounds and/or controlling/directing the synthesis of the desired products. Nevertheless, this will only be possible with the development of efficient methods for modifying a nucleotide sequence at a target site in the genome of the cell of a filamentous fungus, for example, by introducing new genes or sequences for controlling gene expression into the genome of filamentous fungi. This need is addressed by the present invention.

Accordingly the present invention relates in a first aspect to a method for modifying a nucleotide sequence at a target site in the genome of the cell of a filamentous fungus comprising introducing into said cell one or more nucleic molecules comprising a first and a second expression cassette, wherein the first expression cassette comprises a nucleic acid molecule encoding a class 2, type V RNA-guided DNA endonuclease; and wherein the second expression cassette comprises a nucleic acid molecule encoding a DNA-targeting and self-splicing RNA comprising from the 5'-end to the 3'-end (a) a first self-splicing sequence, (b) a stem loop sequence of SEQ ID NO: 7 or 8 or a sequence being at least 75% identical thereto, (c) a nucleotide sequence that is complementary to a sequence at the target site; and (d) a second self-splicing sequence.

The nucleotide sequence at a target site in the genome designates mitochondrial DNA or genomic DNA and preferably genomic DNA. The target site in the genome of the cell (genomic site of interest) is not particularly limited and designates any desired target site in the genome of the cell the nucleotide sequence of which is to be modified. The target site in the genome is preferably a gene of interest (or target gene).

The term "nucleic acid molecule" in accordance with the present invention includes DNA, such as cDNA or double or single stranded genomic DNA and RNA. In this regard, "DNA" (deoxyribonucleic acid) means any chain or sequence of the chemical building blocks adenine (A), guanine (G), cytosine (C) and thymine (T), called nucleotide bases, that are linked together on a deoxyribose sugar backbone. DNA can have one strand of nucleotide bases, or two complimentary strands which may form a double helix structure. "RNA" (ribonucleic acid) means any chain or sequence of the chemical building blocks adenine (A), guanine (G), cytosine (C) and uracil (U), called nucleotide bases, that are linked together on a ribose sugar backbone. RNA typically has one strand of nucleotide bases. Included are also single- and double-stranded hybrid molecules, i.e., DNA-DNA, DNA-RNA and RNA-RNA. The nucleic acid molecule may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Polynucleotides may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphorarnidate linkage. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thio-guanine and fluoro-uracil. A nucleic acid molecule typically carries genetic information, including the information used by cellular machinery to make proteins and/or polypeptides. The nucleic acid molecule of the invention may additionally comprise promoters, enhancers, response elements, signal sequences, polyadenylation sequences, introns, 5'- and 3'- non-coding regions, and the like.

The one or more nucleic acid molecules according to the method of the invention is/are preferably one or more nucleic acid molecule.

The term "expression cassette" designates a distinct component of the one or more nucleic acid molecules consisting of a nuclei acid sequence and a regulatory sequence to be expressed by a transfected cell. The first expression cassette according to the invention encodes a class 2, type V RNA-guided DNA endonuclease and the second expression cassette according to the invention encodes a DNA-targeting and self-splicing RNA. The two expression cassettes direct the cell's machinery to make the class 2, type V RNA-guided DNA endonuclease and the DNA-targeting and self-splicing RNA, respectively.

The term "RNA-guided DNA endonuclease" or "CRISPR(-Cas) endonuclease" describes an enzyme having the capability of cleaving the phosphodiester bond within a deoxyribonucleotide (DNA) strand thereby producing a double-strand break (DSB). An RNA-guided DNA endonuclease comprises an endonuclease domain, in particular a RuvC domain.

An RNA-guided DNA endonuclease also comprises a domain being capable of binding to a crRNA, also known as guide RNA (gRNA; also being designated DNA-targeting RNA herein).

The RNA-guided DNA endonuclease is a class 2, type V RNA-guided DNA endonuclease. The CRISPR-Cas systems are classified into two classes (Classes 1 and 2) that are subdivided into six types (types I through VI). Class 1 (types I, III and IV) systems use multiple Cas proteins in their CRISPR ribonucleoprotein effector nucleases and Class 2 systems (types II, V and VI) use a single Cas protein. Class 1 CRISPR-Cas systems are most commonly found in bacteria and archaea, and comprise ~90% of all identified CRISPR-Cas loci. The Class 2 CRISPR-Cas systems, comprising the remaining ~10%, exists almost exclusively in bacteria, and assemble a ribonucleoprotein complex, consisting of a CRISPR RNA (crRNA) and a Cas protein (Paul and Montoya (2020), Biomedical Journal, 43(1):8-17).

While type II and type V CRISPR-Cas systems each possess a characteristic Ruv-C like nuclease domain, only the RuvC endonuclease domain of type V is made up of three discontinuous parts (RuvC I-III). Type II possesses two nuclease sites HNH and RuvC domains, while type V possesses only one nuclease site in the RuvC domain. Type V comprise a RNase site for processing its own crRNA and this site is situated in the WED-III subdomain. Hence, type V but not type II processes intrinsic RNAse activity to process precrRNA. Type V does not require a tracrRNA or RNase III, since the protein processes its own crRNA in its ribonuclease catalytic site.

The above-discussed features discriminate a class 2, type V RNA-guided DNA endonuclease from all other classes and types of RNA-guided DNA endonucleases.

As discussed herein, above a class 2, type V RNA-guided DNA endonuclease displays an intrinsic RNAse activity that can process a precrRNA into a crRNA in the absence of a tracrRNA. The crRNAs are then incorporated into effector complexes, where the crRNA guides the complex to the invading nucleic acid and the RNA-guided DNA endonucleases to modify the genome at the target site. A guide RNA (gRNA) is an artificially produced piece of RNAs that functions as a naturally-occurring crRNA in that it guides the RNA-guided DNA endonuclease.

The DNA-targeting and self-splicing RNA according to the invention acts like a guide RNA in that it also guides the RNA-guided DNA endonuclease to the target site. However, the DNA-targeting and self-splicing RNA according to the invention is not activated by the intrinsic RNAse activity of a class 2, type V RNA-guided DNA endonuclease, Instead the RNA is capable of self-splicing and splices itself into the active form, wherein it guides the RNA-guided DNA endonuclease to the target site.

Hence, DNA-targeting and self-splicing RNA according to the invention comprises in that order (5' to 3') (a) a first self-splicing sequence, (b) a stem loop sequence of SEQ ID NO: 7 or 8 or a sequence being at least 75% identical thereto, (c) a nucleotide sequence that is complementary to a sequence at the target site; and (d) a second self-splicing sequence.

The first and second self-splicing sequence mediate the self-splicing via sequence-specific intramolecular RNA cleavage. The first and second self-splicing sequence together allow the generation of transcripts with precisely defined ends. The self-splicing liberates the stem loop sequence and the nucleotide sequence.

While the stem loop sequence of SEQ ID NO: 7 or 8 are shown as DNA in the sequence listing they are part of DNA-targeting and self-splicing RNA. Hence, in this context A is replaced by U.

The stem loop sequence and the nucleotide sequence that is complementary to a sequence at the target site are together functionally equivalent to an active guide RNA. The DNA-targeting and self-splicing RNA according to the invention first binds to the class 2, type 5 RNA-guided DNA endonuclease by the stem-loop sequence thereby forming a complex known as CRISPR ribonucleoprotein (RNP) complex. Then the nucleotide sequence that is complementary to a sequence at the target site guides the complex via pairing to a specific location on a DNA strand, where the RNA-guided DNA endonuclease performs its endonuclease activity by cutting the DNA strand at the target site.

The genomic target site can be any about 20 (typically 17 to 26) nucleotide DNA sequence, provided it meets two conditions: (i) The sequence is unique compared to the rest of the genome, and (ii) the target is present immediately adjacent to a Protospacer Adjacent Motif (PAM).

The cleavage site is, thus, furthermore defined by a PAM. The PAM is a short DNA sequence (usually 2-6 base pairs in length) that follows the DNA region targeted for cleavage by the CRISPR system. The exact sequence depends on which CRISPR endonuclease is used. CRISPR endonucleases and their respective PAM sequences are known in the art (see https://www.addgene.org/crispr/guide/#pam-table). For instance, the PAM being recognized by the first identified RNA-guided DNA endonuclease Cas9 is 5'-NGG-3' (where "N" can be any nucleotide base). The PAM is required for a RNA-guided DNA endonuclease to cut. In Cas9 it is found about 2-6 nucleotides downstream of the DNA sequence targeted by the guide RNA and 3-6 nucleotides downstream from the cut site. In type V systems the PAM is located upstream of both, of the target sequence and the cleavage site. The complex of the RNA-guided DNA endonuclease and the guide RNA comprises a so-called PAM interacting domain (Andres et al. (2014), Nature, 513(7519):569-573). Hence, the genomic locations that can be targeted for editing by an RNA-guided DNA endonuclease are limited by the presence and locations of the nuclease-specific PAM sequence. Class 2, type V CRISPR nucleases display a T-rich PAM site (preferably TTTN, wherein N can be any nucleotide of A, T, C and G).

The stem loop sequence according to the invention comprises or consists of SEQ ID NO: 7 or 8, or a sequence being at least 75% identical thereto. The term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 80% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Amino acid sequence as well as nucleotide sequence analysis and alignments in connection with the present invention are preferably carried out using the NCBI BLAST algorithm (Stephen F. Altschul, Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402). The skilled person is aware of additional suitable programs to align nucleic acid sequences.

The above at least 75% sequence identity is with increasing preference at least 80%, at least 85%, at least 90%, and at least 95% and 100%.

The modification of a nucleotide sequence at a target site in a genome is also referred to herein as genome editing (also known as genome engineering). Genome editing is a type of genetic engineering in which a target site in a genome is modified, for example, by the insertion in, the deletion of, or the replacement of a target gene of interest is in the genome of the cell. As will be further detailed herein below, genome editing uses the cell's own repair pathways, including the non-homologous end-joining (NHEJ) or homology directed recombination (HDR) pathway. It is preferred that genome editing uses NHEJ. Genome editing via NHEJ is illustrated in the examples. In a different embodiment, it is preferred that genome editing uses HDR. Genome editing may result in a loss-of-function mutation or a gain-of-function mutation in the genome of the cell. A loss-of-function mutation (also called inactivating mutation) results in the gene of interest having decreased or no function (being partially or wholly inactivated). When the allele has a complete loss of function (wholly inactivated) this is also called herein a (gene) knock-out. Genome editing of the gene of interest is preferably a knock-out. A gene knock-out may be achieved by inserting, deleting, modifying or replacing one or more nucleotides of a gene. A gain-of-function mutation (also called activating mutation) may change the gene of interest such that its effect gets stronger (enhanced activation) or even is superseded by a different (e.g. abnormal) function. A gain-of-function mutation may also introduce a new function or effect into a cell which the cell did not have before. In this context the new gene may be added to the genome of the cell (insertion) or may replace a gene within the genome. A gain-of-function mutation introducing such a new function or effect is also called gene knock-in.

Genome editing uses in accordance with the invention the nucleases of the clustered regularly interspaced short palindromic repeats (CRISPR/Cas) system (also called herein CRISPR nucleases). CRISPR nucleases (or CRISPR-Cas nucleases or Cas nucleases) are a specific type of programmable nucleases and the CRISPR nuclease to be used in in accordance with the invention a class 2, type V RNA-guided DNA endonuclease, as discussed herein above.

A filamentous fungus is a fungus being capable of forming a filamentous structure known as hyphae. Filamentous structures are multicellular structures with branching. Most of these hyphae extend in 3 dimensions. Specialised hyphae are produced to allow vegetative (non-sexual) reproduction with spores or conidia. Some highly specialised reproductive or protective structures are also formed by some species, such as ascospores. There are probably millions of species in total. Filamentous fungi are found in many phylogenetic groups, but the vast majority of human pathogens are Ascomycetes.

Means for introducing DNA molecules into living cells are known in the art and comprise but are not limited to transfection and transduction.

Transduction is the process by which foreign DNA is introduced into a cell by a virus or viral vector. Transduction is a common tool used by molecular biologists to stably introduce a foreign gene into a host cell's genome. Generally, a plasmid is constructed in which the genes to be transferred are flanked by viral sequences that are used by viral proteins to recognize and package the viral genome into viral particles. This plasmid is inserted (usually by transfection) into a producer cell together with other plasmids (DNA constructs) that carry the viral genes required for formation of infectious virions. In these producer cells, the viral proteins expressed by these packaging constructs bind the sequences on the DNA/RNA (depending on the type of viral vector) to be transferred and insert it into viral particles. For safety, none of the plasmids used contains all the sequences required for virus formation, so that simultaneous transfection of multiple plasmids is required to get infectious virions. Moreover, only the plasmid carrying the sequences to be transferred contains signals that allow the genetic materials to be packaged in virions, so that none of the genes encoding viral proteins are packaged. Viruses collected from these cells are then applied to the cells to be altered. The initial stages of these infections mimic infection with natural viruses and lead to expression of the genes transferred and (in the case of lentivirus/retrovirus vectors) insertion of the DNA to be transferred into the cellular genome. However, since the transferred genetic material does not encode any of the viral genes, these infections do not generate new viruses (the viruses are "replication-deficient").

Transfection is the process of deliberately introducing naked or purified nucleic acids or purified proteins or assembled ribonucleoprotein complexes into cells. Transfection is generally a non-viral based method.

Transfection may be a chemical-based transfection. Chemical-based transfection can be divided into several kinds: transfection using cyclodextrin, polymers, liposomes, or nanoparticles. One of the cheapest methods uses calcium phosphate. HEPES-buffered saline solution (HeBS) containing phosphate ions are combined with a calcium chloride solution containing the DNA to be transfected. When the two are combined, a fine precipitate of the positively charged calcium and the negatively charged phosphate will form, binding the DNA to be transfected on its surface. The suspension of the precipitate is then added to the cells to be transfected (usually a cell culture grown in a monolayer). By a process not entirely understood, the cells take up some of the precipitate, and with it, the DNA. This process has been a preferred method of identifying many oncogenes. Other methods use highly branched organic compounds, so-called dendrimers, to bind the DNA and transfer it into the cell. Another method is the use of cationic polymers such as DEAE-dextran or polyethylenimine (PEI). The negatively charged DNA binds to the polycation and the complex is taken up by the cell via endocytosis. Lipofection (or liposome transfection) is a technique used to inject genetic material into a cell by means of liposomes, which are vesicles that can easily merge with the cell membrane since they are both made of a phospholipid bilayer. Lipofection generally uses a positively charged (cationic) lipid (cationic liposomes or mixtures) to form an aggregate with the negatively charged (anionic) genetic material. This transfection technology performs the same tasks in terms of transfer into cells as other biochemical procedures utilizing polymers, DEAE-dextran, calcium phosphate, and electroporation. The efficiency of lipofection can be improved by treating transfected cells with a mild heat shock. Fugene is a series of widely used proprietary non-liposomal transfection reagents capable of directly transfecting a wide variety of cells with high efficiency and low toxicity.

Transfection may also be a non-chemical method. Electroporation (gene electrotransfer) is a popular method, where transient increase in the permeability of cell membrane is achieved when the cells are exposed to short pulses of an intense electric field. Cell squeezing enables delivery of molecules into cells via cell membrane deformation. Sonoporation uses high-intensity ultrasound to induce pore formation in cell membranes. This pore formation is attributed mainly to the cavitation of gas bubbles interacting with nearby cell membranes since it is enhanced by the addition of ultrasound contrast agent, a source of cavitation nuclei. Optical transfection is a method where a tiny (~1 µm diameter) hole is transiently generated in the plasma membrane of a cell using a highly focused laser. Protoplast fusion is a technique in which transformed bacterial cells are treated with lysozyme in order to remove the cell wall. Following this, fusogenic agents (e.g., Sendai virus, PEG, electroporation) are used in order to fuse the protoplast carrying the gene of interest with the recipient target cell.

Finally, transfection may be a particle-based method. A direct approach to transfection is the gene gun, where the DNA is coupled to a nanoparticle of an inert solid (commonly gold), which is then "shot" (or particle bombardment) directly into the target cell's nucleus. Hence, the nucleic acid is delivered through membrane penetration at a high velocity, usually connected to microprojectiles. Magnetofection, or magnet-assisted transfection, is a transfection method that uses magnetic force to deliver DNA into target cells. Impalefection is carried out by impaling cells by elongated nanostructures and arrays of such nanostructures such as carbon nanofibers or silicon nanowires which have been functionalized with plasmid DNA.

As is evident from the appended examples experiments carried out in filamentous fungi showed that classical gRNA expression cassettes using RNA polymerase III promoters commonly used with classical CRISPR nucleases such as Cas9 did not lead to an acceptable genome editing activity. Therefore, a novel gRNA expression cassette with specific gRNA processing elements was developed to enable efficient class 2, type V RNA-guided DNA endonuclease-driven genome editing in filamentous fungi. The examples also show that the gRNA expression cassette particularly well works with the BEC family of class 2, type V RNA-guided DNA endonucleases. BEC family RNA-guided DNA endonucleases are a novel type of CRISPR nucleases with already proven genome editing activity in E. coli and various prokaryotic and eukaryotic organisms (PCT/EP2021/000081).

In accordance with a preferred embodiment of the method of the first aspect of the invention the nucleic acid molecule encoding a class 2, type V RNA-guided DNA endonuclease is (a) a nucleic acid molecule encoding the amino acid sequence of SEQ ID NO: 1, 2 or 3; (b) a nucleic acid molecule comprising or consisting of the nucleotide sequence of SEQ ID NO: 4, 5, 6 or 16; (c) a nucleic acid molecule encoding a RNA-guided DNA endonuclease the amino acid sequence of which is at least 80 % identical to the amino acid sequence of (a); (d) a nucleic acid molecule comprising or consisting of a nucleotide sequence which is at least 80 % identical to the nucleotide sequence of (b); or (e) a nucleic acid molecule which is degenerate with respect to the nucleic acid molecule of (d).

The amino acid sequence of SEQ ID NO: 1, 2 or 3 and the nucleotide sequence of SEQ ID NO: 4, 5 or 6 are the amino acid sequences and the nucleotide sequences of the class 2, type V RNA-guided DNA endonucleases of BEC85, BEC67 and BEC10 as disclosed in the international application PCT/EP2021/000081. The nucleotide sequence of SEQ ID NO: 16 is the nucleotide sequence of the class 2, type V RNA-guided DNA endonucleases of BEC10 that has been optimized for the expression in *Aspergillus niger.* SEQ ID NO: 16 is therefore also designated AspBEC10 herein.

It is shown in PCT/EP2021/000081 that the activity profile of BEC family members is completely different compared to the activity profile of other known CRISPR nucleases. The endonuclease activity of BEC85, BEC67 and BEC10, respectively, is based on a novel molecular mechanism which has not been described before. For this reason, BEC85, BEC67 and BEC10 can be classified as novel, non-naturally occurring class 2, type V nucleases with overall no significant sequence identity to the known collection of class 1 and class 2 CRISPR-Cas endonucleases and with overall low sequence identity to individual Cms1-type endonucleases.

BEC85, BEC67 and BEC10 are CRISPR-Cas endonucleases which are significantly distinct from the known collection of CRISPR-Cas endonucleases and are showing a novel mechanism of activity, BEC85, BEC67 and BEC10 expand the known collection of CRISPR-Cas endonucleases applicable for genome editing, gene regulation and nucleic acid enrichment/purification in different biotechnological and pharmaceutical sectors. The results in PCT/EP2021/000081 strongly indicate that BEC type CRISPR nucleases are not only a novel type of effector proteins with distinct locus architectures but also display a new molecular genome editing mechanism.

It is furthermore demonstrated in in PCT/EP2021/000081 that genome editing using the BEC family type nucleases provide for significantly higher clone reduction numbers and significantly superior editing ratios as compared to their next neighbor sequences SuCms1 (Begemann et al. (2017), bioRxiv) and SeqID63 (WO 2019/030695). The results further prove the general superiority of the BEC type nucleases for genome editing as compared to the previously known CRISPR Cas nucleases.

Yet further, it is demonstrated in in PCT/EP2021/000081 that the novel BEC family type nucleases display strong activity at temperature levels from 21°C to 37°C and in particular a superior genome editing efficiency and colony reduction rate as compared to the next neighbor sequences SuCms1 and SeqID63. For instance, the genome editing efficiency of the SuCms1 nuclease significantly decreases at 21°C to levels comparable to the negative control (0.3 %) whereas the BEC10 editing efficiency remains at a high level (65 %) even at the relative low temperature of 21°C. High activity within a temperature range from 21°C to 37°C is of great interest for biotechnological, agricultural and pharmaceutical applications because within this temperature range various types of cells are cultured (e.g. various plants and plant cells ≈ 21°C, various yeast and fungal cells ≈ 30°C, various prokaryotic organisms and mammalian cell lines ≈ 37°C). The novel BEC family type nucleases therefore advantageously allow the design of universally applicable CRISPR systems.

The sequence identities of at least 80 % in items (c) and (c) in the above preferred embodiment are with increasing preference (for each items separately) at least 80 %, at least 81 %, at least 82 %, at least 83 %, at least 84 %, at least 85 %, at least 86 %, at least 87 %, at least 88 %, at least 89 %, at least 90 %, at least 91 %, at least 92 %, at least 93 %, at least 94 %, at least 95 %, at least 96 %, at least 97 %, at least 98 %, and at least 99 %.

The term "degenerate" designates the degeneracy of the genetic code. As is well known, the codons encoding one amino acid may differ in any of their three positions; however, more often than not, this difference is in the second or third position. For instance, the amino acid glutamic acid is specified by GAA and GAG codons (difference in the third position); the amino acid leucine is specified by UUA, UUG, CUU, CUC, CUA, CUG codons (difference in the first or third position); and the amino acid serine is specified by UCA, UCG, UCC, UCU, AGU, AGC (difference in the first, second, or third position).

In accordance with another preferred embodiment of the method of the first aspect of the invention the class 2, type V RNA-guided DNA endonuclease is selected from Cpf1 (Cas12a), C2c1 (Cas12b), Cas12c, Cas12d, Cpf1 (Cas12a), Cms1 (Cas12e), Cas12g, Cas12h, Cas12i, Cas12k, Cas12j and Cas14.

While the BEC family members are related to the preferred class 2, type V RNA-guided DNA endonuclease when performing the method of the invention, the invention is not limited to the of BEC family members but in general any class 2, type V RNA-guided DNA endonuclease may be used. Cpf1 (Cas12a), C2c1 (Cas12b), Cas12c, Cas12d, Cpf1 (Cas12a), Cms1 (Cas12e), Cas12g, Cas12h, Cas12i, Cas12k, Cas12j and Cas14 are non-limiting examples of known class 2, type V RNA-guided DNA endonucleases.

In accordance with another preferred embodiment of the method of the first aspect of the invention, the method further comprises culturing the cell under conditions, wherein the RNA-guided DNA endonuclease and DNA-targeting and self-splicing RNA are expressed in the cell.

As discussed above, in the method of the invention one or more nucleic molecules comprising a first and a second expression cassette are introduced into the cell of a filamentous fungus. The expression of the sequences being encoded by the first and a second expression cassette in the cell can be favored by selecting appropriate cell culturing conditions.

Since filamentous fungi are widely used for the production of many industrially relevant compounds, such as organic acids, proteins, enzymes, exopolysaccharides, and secondary metabolites appropriate cell culturing conditions are well known in the art; see, for example, Cairns et al. (2019), Biotechnology for Biofuels, 12:77 or Vrabl et al.(2019), Front. Microbiol., 10:2391.

The present invention relates in second aspect to the use of one or more nucleic molecules comprising a first and a second expression cassette as defined in connection with the first aspect of the invention for modifying a nucleotide sequence at a target site in the genome of a cell of a filamentous fungus.

The definitions and preferred embodiments of the first aspect of the invention apply *mutatis mutandis* to the second aspect of the invention, as far as being applicable to the second aspect of the invention.

In accordance with a preferred embodiment of the first and second aspect of the invention, the expression of the second expression cassette is under the control of a constitutive or an inducible promoter, preferably a RNA polymerase II promoter and most preferably the gpdA RNA polymerase II promoter.

A promoter is a sequence of DNA to which proteins bind that initiate downstream transcription of a single RNA from the DNA. A constitutive promoter is transcriptionally active in all circumstances in the cell. A constitutive promoter has to be held distinct from am inducible promotor that only becomes active in the cell in response to a specific stimulus.

A RNA polymerase II promoter (RNA pol II) is a multiprotein complex that transcribes DNA into precursors of messenger RNA (mRNA) and most small nuclear RNA (snRNA) and microRNA. mRNA pol II transcripts comprise a cap structure and a poly (A)-tail.

Since the cap structure and the poly (A)-tail block the processing of the guide RNA from forming an RNP with the RNA-guided DNA, endonuclease RNA pol II is not suitable for classical CRISPR cas genome editing. For classical CRISPR cas genome editing usually RNA poll III promoters are used that normally transcribe tRNA genes and do not form the cap structure and the poly (A)-tail.

Since the DNA-targeting and self-splicing RNA according to the invention splices itself into a DNA-targeting RNA being functionally equivalent to a guide RNA it is possible to use an RNA pol II in the context of the present invention. This is because the cap and the poly (A) are cleaved-off together with the auto-splicing thereby releasing a functional equivalent to a classical guide RNA.

The gpdA RNA polymerase II promoter as used in the examples is shown in SEQ ID NO: 20. The gpdA RNA polymerase II promoter to be used shares with increasing preference at least 80%, at least 85%, at least 90%, at least 95% and 100% sequence identity with SEQ ID NO: 20.

In accordance with a further preferred embodiment of the first and second aspect of the invention, the expression from the second expression cassette is under the control of a terminator, preferably a trpC terminator (TtrpC)

The terminator defines the end of a transcriptional unit (such as a gene) and initiates the process of releasing the newly synthesized RNA from the transcription machinery. Terminators are found downstream of the sequence to be transcribed, and typically occur directly after any 3' regulatory elements, such as the polyadenylation or poly(A) signal. While many studies focus on promoter strength as a determinant of gene expression levels, the terminator also plays an important role in RNA processing and contributes to variability in RNA half-life, and ultimately gene-expression.

The trpC terminator is used in the examples herein below and is particularly useful for filamentous fungi, such as *Aspergillus nidulans* or *niger.*

The trpC terminator sequence as used in the appended examples is shown in SEQ ID NO: 12. The trpC terminator sequence to be used shares with increasing preference at least 80%, at least 85%, at least 90%, at least 95% and 100% sequence identity with SEQ ID NO: 12.

In accordance with another further preferred embodiment of the first and second aspect of the invention, the first self-splicing sequence comprises at the 3'-end a nucleotide sequence that base-pairs with the stem loop sequence of (b).

This base-pairing is advantageous in connection with certain ribozymes, in particular the hammerhead (HH) ribozyme since it promotes efficient cleavage. If follows that in connection with this preferred embodiment the self-splicing sequences are ribozymes and preferably one self-splicing sequence is the hammerhead (HH) ribozyme. Further details on ribozymes and the hammerhead (HH) ribozyme will be provided herein below.

In accordance with a further preferred embodiment of the first and second aspect of the invention, the filamentous fungus is selected from *Aspergillus spp*., *Trichoderma spp*, *Penicillium spp*., *Fusarium* spp., *Claviceps* spp., *Hypomyces* spp., *Paecilomyces* spp., *Talaromyces* spp. and *Neurospora spp*., wherein the *Aspergillus spp.* is preferably selected from *Aspergillus niger*, *Aspergillus nidulans*, *Aspergillus fumigatus*, *Aspergillus oryzae*, *Aspergillus flavus* and *Aspergillus terreus* and is preferably *Aspergillus niger.*

While a plethora of filamentous fungi is known and the genome of many filamentous fungi is already sequenced (1000 Fungal Genomes Project), in particular the filamentous fungi are preferred since they are used in the art as production organisms in biotechnology.

In accordance with a yet further preferred embodiment of the first and second aspect of the invention, the nucleic acid molecule encoding the RNA-guided DNA endonuclease is codon-optimized for the expression in a filamentous fungus, preferably *Aspergillus niger.*

There are 64 different codons that encode 20 amino acids and three stop codons. This means that the same amino acid can be encoded by more than one codon. Although the genetic code is universal, different organisms prefer certain codons over others for certain amino acids. This is termed codon usage bias. Codon optimization is an approach in gene engineering to improve gene expression by changing synonymous codons based on an organism's codon bias. Mutations are made throughout a gene of interest based on an organism's codon usage bias to increase translational efficiency and thus protein expression without altering the sequence of the protein.

The codon optimization approach is well-established and even software for automated codon optimization is available.

In accordance with another preferred embodiment of the first and second aspect of the invention, the first expression cassette the nucleic acid molecule encoding a RNA-guided DNA endonuclease is extended at the 5'-end and/or the 3'-end by a nuclear localisation signal (NLS), wherein the 5'-NLS is preferably the c-myc NLS and/or the 3'-NLS is preferably the nucleoplasmin NLS and/or one or two SV40 NLS.

The first expression cassette encodes the class 2, type V RNA-guided DNA endonuclease according to the invention. An NLS is an amino acid sequence that 'tags' a protein for import into the cell nucleus by nuclear transport. Typically, this signal consists of one or more short sequences of positively charged lysines or arginines exposed on the protein surface.

The NLS therefore helps to transport class 2, type V RNA-guided DNA endonucleases into the nucleus where it cuts the DNA at the target site.

The amino acid sequences of the c-myc NLS, the nucleoplasmin NLS and the SV40 NLS are shown in SEQ ID NOs 13 to 15. Each NLS to be used shares with increasing preference at least 80%, at least 85%, at least 90%, at least 95% and 100% sequence identity with one of SEQ ID NOs 13 to 15.

In accordance with an additional preferred embodiment of the first and second aspect of the invention, the expression from the first expression cassette is under the control of a constitutive or inducible promoter, preferably an *Aspergillus ssp.* tef1 promoter and most preferably an *Aspergillus nidulans* tef1 promoter.

The tef (translation elongation factor) 1 promoter sequence as used in the appended examples is shown in SEQ ID NO: 11. The tef1 promoter sequence to be used shares with increasing preference at least 80%, at least 85%, at least 90%, at least 95% and 100% sequence identity with SEQ ID NO: 11. An Aspergillus ssp. tef1 promoter has a strong promoter activity and is therefore preferred.

The tef1 promoter sequence is preferably used together with the tef1 terminator sequence. The tef1 terminator sequence as used in the appended examples is shown in SEQ ID NO: 19. The tef1 terminator sequence to be used shares with increasing preference at least 80%, at least 85%, at least 90%, at least 95% and 100% sequence identity with SEQ ID NO: 19.

In accordance with a further preferred embodiment of the first and second aspect of the invention, the one or more nucleic molecules are or are expressed in one or two expression vectors and preferably in one expression vector.

In accordance with a more preferred embodiment of the first and second aspect of the invention, the expression vector(s) is/are *E. coli*/*A. niger* shuttle vector(s), wherein the shuttle vector(s) is/are preferably capable of episomal propagation in *E. coli* and *A. niger* cells and/or preferably comprise(s) one or more markers for the selection of recombinant *E. coli* and/or A. *niger* cells.

The one or more nucleic acid molecules are preferably one or more vectors, and preferably one vector.

Preferably, the one or more vectors can be plasmids, cosmids, viruses, bacteriophages or other vectors used conventionally e.g. in genetic engineering.

The nucleic acid molecules used in accordance with the present invention may be inserted into several commercially available vectors. Single vectors containing both the CRISPR endonuclease and the gRNAs are commercially available, thereby acting as an all-in-one vector. The method of the invention can alternatively be implemented by using two or three vectors containing the CRISPR endonuclease and the at least two gRNAs. It is also possible to use gRNA-only vectors and use cells in which the CRISPR endonuclease has been integrated into the genome. The use of an all-in-one vector that expresses the at least two gRNAs and the CRISPR endonuclease is preferred since only one vector is to be introduced into the cells. A vector which can express the CRISPR endonuclease and up to seven gRNAs is, for example, described in Sakuma et al, Sci Rep. 2014; 4: 5400.

Many single gRNA empty vectors (with and without the CRISPR endonuclease) are available in the art. Likewise several empty multiplex gRNA vectors are available that can be used to express multiple gRNAs from a single plasmid (with or without the expression of the CRISPR endonuclease). Finally, also vectors are available that only express the CRISPR endonuclease (see https://www.addgene.org/crispr/empty-grna-vectors/).

Vector modification techniques are known in the art and, for example, described in Sambrook and Russel, 2001. Generally, vectors can contain one or more origins of replication (ori) and inheritance systems for cloning or expression, one or more markers for selection in the host, e.g., antibiotic resistance, and one or more expression cassettes. Suitable origins of replication include, for example, the Col E1, the SV40 viral and the M 13 origins of replication. The nucleic acid sequences inserted in the vector can e.g. be synthesized by standard methods, or isolated from natural sources. Ligation of the coding sequences to transcriptional regulatory elements and/or to other amino acid encoding sequences can be carried out using established methods. Such regulatory sequences are well known to those skilled in the art and include, without being limiting, regulatory sequences ensuring the initiation of transcription, initiation of translation, internal ribosomal entry sites (IRES) or 2A linker (Owens, Proc. Natl. Acad. Sci. USA 98 (2001), 1471-1476) and optionally regulatory elements ensuring termination of transcription and stabilization of the transcript. Non-limiting examples for regulatory elements ensuring the initiation of transcription comprise a translation initiation codon, enhancers such as e.g. the SV40-enhancer, insulators and/or promoters, such as for example the cytomegalovirus (CMV) promoter, elongation factor-1 alpha (EF1-alpha), promoter, SV40-promoter, RSV-promoter (Rous sarcoma virus), the lacZ promoter, chicken beta-actin promoter, CAG-promoter (a combination of chicken beta-actin promoter and cytomegalovirus immediate-early enhancer), the gai10 promoter, human elongation factor 1a-promoter, AOX1 promoter, GAL1 promoter, CaM-kinase promoter, the lac, trp or tac promoter, the lacUV5 promoter, the *Autographa californica* multiple nuclear polyhedrosis virus (AcMNPV) polyhedral promoter or a globin intron in mammalian and other animal cells. Non-limiting examples for regulatory elements ensuring transcription termination include the SV40-poly-A site, the tk-poly-A site or the SV40, lacZ or AcMNPV polyhedral polyadenylation signals, which are to be included downstream of the nucleic acid sequence of the invention. Additional regulatory elements may include translational enhancers, Kozak sequences and intervening sequences flanked by donor and acceptor sites for RNA splicing, nucleotide sequences encoding secretion signals or, depending on the expression system used, signal sequences capable of directing the expressed polypeptide to a cellular compartment. Moreover, elements such as origin of replication, drug resistance gene, regulators (as part of an inducible promoter) may also be included.

In accordance with a preferred embodiment of the first and second aspect of the invention,, the first and/or the second self-splicing sequence are preferably a ribozyme or tRNA, wherein the ribozyme is preferably selected from the group of a hepatitis delta virus (HDV) ribozyme, hammerhead (HH) ribozyme, hairpin (HP) ribozyme, glucosamine-6-phosphate riboswitch (glmS) ribozyme, type-P1 twister ribozyme from rice, and varkud satellite (VS) ribozymes. The first and/or the second self-splicing sequence are most preferably a hepatitis delta virus (HDV) ribozyme and a hammerhead (HH) ribozyme.

The sequences of the hepatitis delta virus (HDV) ribozyme and the hammerhead (HH) ribozyme as used in the examples are shown in SEQ ID NOs 9 and 10. The hepatitis delta virus (HDV) ribozyme and a hammerhead (HH) ribozyme used share each independently with increasing preference at least 80%, at least 85%, at least 90%, at least 95% and 100% sequence identity with SEQ ID NOs 9 and 10.

The use of the hammerhead (HH) ribozyme is particularly advantageous in connection with Class 2 Type V nucleases including the BEC family nucleases as compared to the classical CRISPR Cas nucleases including Cas 9.

For the reasons explained in the appended examples, for classical CRISPR Cas nucleases including Cas 9 the HH sequence needs to be adjusted to match the spacer sequences whereas this is not necessary for Class 2 Type V nucleases including the BEC family nucleases. The same HH sequence can be used irrespective of the spacer sequence. This is an important advantage since the spacer sequence determines the target specificity of a CRISPR Cas nuclease,

As used herein, the term "ribozyme" designates an RNA molecule with catalytic activity, i.e. the chemical nature is ribonucleic acid (RNA), but a ribozyme has the catalytic function of an enzyme. The active substrate of the ribozyme may be a different molecule. Compared with a protein enzyme, the ribozyme is low in catalytic efficiency, yet breaking the traditional concept of enzymes as proteins. Various ribozymes with self-cleaving activity are known from the prior art and can be used herein.

While ribozymes are preferred, instead of ribozymes also tRNA self-splicing elements may be used (Vanegas et al. (2019), Fungal Biology and Biotechnology; 6:6). In bacteria tRNA introns are self-spliced and such tRNA self-splicing elements may also be employed herein.

The present invention relates in a third aspect to one or more nucleic acid molecules and preferably one nucleic acid molecule comprising a first and a second expression cassette wherein the first expression cassette comprises a nucleic acid molecule encoding a class 2, type V RNA-guided DNA endonuclease as defined in connection with the first aspect of the invention; and wherein the second expression cassette comprises a nucleic acid molecule encoding a DNA-targeting and self-splicing RNA comprising from the 5'-end to the 3'-end (a) a first self-splicing sequence, (b) a stem loop sequence of SEQ ID NO: 7 or 8 or a sequence being at least 75% identical thereto, (c) a cloning site into which a nucleotide sequence that is complementary to a sequence at the target site can be added; and (d) a second self-splicing sequence.

The present invention relates in a fourth aspect to a kit for modifying a nucleotide sequence at a target site in the genome of a cell, preferably of a cell of a filamentous fungus, wherein the kits comprises one or more nucleic molecules comprising a first and a second expression cassette wherein the first expression cassette comprises a nucleic acid molecule encoding a class 2, type V RNA-guided DNA endonuclease as defined in connection with the first aspect of the invention; and wherein the second expression cassette comprises a nucleic acid molecule encoding a DNA-targeting and self-splicing RNA comprising from the 5'-end to the 3'-end (a) a first self-splicing sequence, (b) a stem loop sequence of SEQ ID NO: 7 or 8 or a sequence being at least 75% identical thereto, (c) a cloning site into which a nucleotide sequence that is complementary to a sequence at the target site can be added; and (d) a second self-splicing sequence, wherein the kit optionally further comprises one or more of (i) cells, preferably cells of a filamentous fungus, (ii) a medium for culturing cells and/or the expression of the RNA-guided DNA endonuclease and the DNA-targeting and self-splicing RNA, (iii) instructions for using the kit for modifying a nucleotide sequence at a target site in the genome of a cell, preferably of a cell of a filamentous fungus.

The definitions and preferred embodiments of the first and second aspect of the invention apply *mutatis mutandis* to the third and fourth aspect of the invention, as far as being applicable to the third and fourth aspect of the invention.

The components of the kit can be packed separately or in different combinations, taking into account the intended use for modifying a nucleotide sequence at a target site in the genome of a cell. The components of the kit can be packed, for example, into vials, tubes or bags.

The medium for culturing cells and/or the expression of the RNA-guided DNA endonuclease and the DNA-targeting and self-splicing RNA can be one medium wherein the cells grow and express the RNA-guided DNA endonuclease and the DNA-targeting and self-splicing RNA. Also two different media can be comprised in the kit, wherein one medium favors growth and the other medium favors expression.

The instructions can be in the format of a leaflet in the package or can also be in the format of a weblink, barcode or QR code on the package.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show.

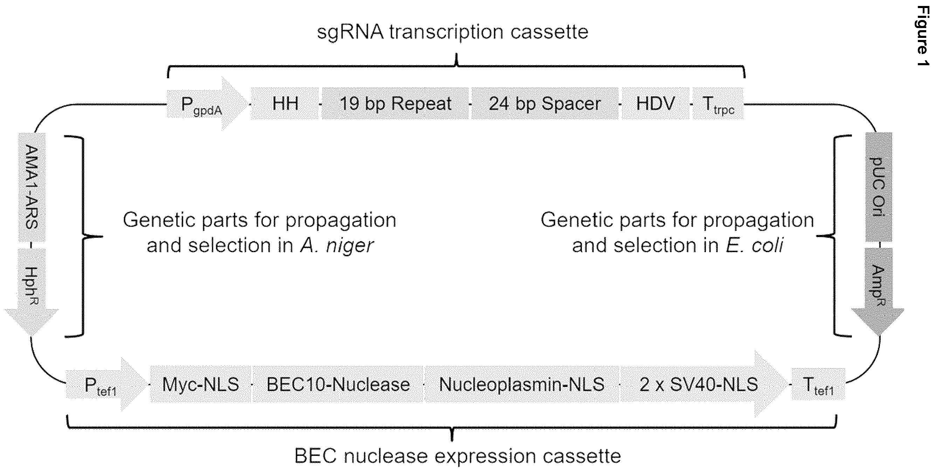

**Figure 1** - Map of the CRISPR/AspBEC10 all-in-one vector system
**Figure 2** - CRISPR/AspBEC10 all-in-one system containing a gRNA against the chromosomal ku70-gene resulted in a strongly reduced number of viable cells in the NHEJ deficient *ku70⁻mutant* strain variant of *Aspergillus niger* WBT-AN-011 (CBS 141599).
**Figure 3** - Protoplast-mediated co-transformation of NHEJ deficient strain *Aspergillus niger* WBT-AN-011 (CBS 141599) with 1.5 µg of the programmed CRISPR/AspBEC10 plasmid and 1.5 µg of the HDR-template resulted in selection of 3 recombinant clones of large size that gave fluorescent signals stemming from functional *gfp* integrated into the *ku70* locus of the *Aspergillus niger* strain.

The Examples illustrate the invention.

### Example 1: Construction of a functional genome editing system for BEC family proteins in Aspergillus niger

### Introduction - CRISPR/AspBEC10 all-in-one vector system for genome editing in Aspergillus niger

To demonstrate the successful genome editing of filamentous fungi using BEC family nucleases and a specifically designed gRNA expression setup, an all-in-one vector system containing the BEC10 nuclease and a specifically designed gRNA expression cassette was generated. Some elements of the basic vector architecture of the constructed CRISPR/AspBEC10 all-in-one plasmid system consist of genetic parts which were already used for CRISPR-mediated mutagenesis of filamentous fungi and has been described exemplarily by the publication of Nødvig et al. (2015).

In comparison to the vector system described by Nødvig et al. (2015), the vector architecture has been further developed and optimized herein. This has several advantages for easier vector adaptation and the expression of the nuclease and the gRNA. For example, the integration of a spacer sequence to target a specific region on the genome is significantly more convenient to perform, as the HH ribozyme sequence does not have to be adjusted to the spacer sequence. The nucleases used by Nødvig et al. (2015) require gRNAs where the adaptable spacer sequence is located on the 5' end of the gRNA. As the 5' prime end of the HH sequence base pairs with the 5' end of the gRNA (where the spacer sequence is located), the HH sequence needs to be adjusted to match the spacer sequences (the spacer sequence is different in every experimental approach with regard to the target region in the genome). In contrast, the spacer of the Class 2 Type V nucleases including the BEC family nucleases is located on the 3' prime end of the gRNA. Therefore, the 5' prime end of the gRNA consists of the constant stem loop sequence that remains unaltered regardless of the respective spacer sequences thus simplifying the process to design and introduce new spacer sequences significantly and providing a technical advantage over the vector system of Nødvig et al (2015).

In further experiments, the vector was adapted and optimized for successful application of the BEC family nucleases by combining various *in vivo* strategies for gRNA processing and provision. Finally, a defined strategy to deliver and process a functional gRNA resulted in dramatic clone reduction and a strong genome editing activity induced by the BEC10 nuclease.

In the following, the construction of the functional CRISPR/AspBEC10 all-in-one vector system for Genome editing in *Aspergillus niger* is described.

### DNA techniques

If not mentioned otherwise, plasmid isolation, enzymatic manipulation of DNA and agarose gel electrophoresis were performed according to standard procedures as described by Green & Sambrook, Molecular Cloning: A Laboratory Manual (2012). The Thermo Fisher Scientific Phusion Flash High-Fidelity PCR system (Thermo Fisher Scientific, Darmstadt, Germany) was used for PCR amplifications according to the manufacture's manual. All oligonucleotides used in this work were synthesized by biomers.net (Ulm, Germany) or Eurofins Scientific (Ebersberg, Germany). The identity of all cloned DNA-fragments was confirmed by Sanger sequencing technology at LGC Genomics (Berlin, Germany).

### Transformation of E. coli cells

Commercial available NEB 5-alpha or NEB 10-beta competent *E. coli* cells (New England Biolabs, Frankfurt, Germany) were used for transformation of ligated DNA fragments, Gibson assembly reactions (New England Biolabs, Frankfurt, Germany) and plasmid DNA. The transformation procedure and selection of recombinant *E. coli* cells followed the manufacturer's high efficiency transformation protocol.

### Aspergillus niger WBT-AN-011 (CBS 141599) cultivation and transformation

The protoplast-mediated transformation of *Aspergillus niger* WBT-AN-011 (CBS 141599) was performed according to the protocol by Arentshorst et al. Methods Mol Biol (2012) 835: 133 - 150. In brief, *Aspergillus niger* WBT-AN-011 (CBS 141599) was incubated for 120 hours in 100 ml complete medium at 30°C and 150 rpm on a horizontal shaker. The mycelium was harvested by filtration through a sterile paper filter and washed with SMC buffer. To 1 g mycelium the protoplastation solution was added and the suspension was incubated for two hours at 37°C and 80 rpm on a horizontal shaker. The protoplasts were collected through a sterile myracloth filter, pelleted, washed and resuspended in STC solution. For the transformation procedure 2 µg plasmid DNA, 100 µl protoplasts and 25 µl PEG buffer were gently mixed. Afterwards, 1 ml PEG buffer was added and the mixture was incubated for exactly 5 minutes at room temperature. The transformation reaction was mixed with 12.5 ml MMS top agar containing caffeine and hygromycin. The top agar was poured onto MMS selection agar plates and incubated for one week at 30°C.

### Genetic analysis of recombinant Aspergillus niger clones - Rapid diagnostic PCR analysis of recombinant Aspergillus niger clones

For fast genetic screening of recombinant *Aspergillus niger* clones, colony PCR was performed using cell material instead of purified genomic DNA as template for the amplification reaction. Cells were resuspended in water and mixed with the prepared PCR cocktail containing specific primer. The initial denaturation step of the cycle program was extended to 10 minutes, to ensure cell lysis and liberation of genomic DNA.

### Extraction of genomic DNA from Aspergillus niger cells

Extraction of purified genomic DNA from *Aspergillus niger* cells was conducted as described by Arentshorst et al. Methods Mol Biol (2012) 835: 133 - 150. In brief, mycelium was harvested by vacuum filtration and homogenized in liquid nitrogen. After removal of cell wall components, interfering proteins and RNA, the genomic DNA was extracted from the aqueous phase with phenol:chloroform:isoamyl alcohol and precipitated with isopropanol. Finally, the purified genomic DNA was washed with 70 % ethanol and resuspended in ultrapure water.

### Design of the AspBEC10 protein expression cassette

The synthetic 3693 bps BEC10 nucleotide sequence was codon optimized for expression in *Aspergillus niger* (AspBEC10), using a bioinformatics application provided by the gene synthesis provider GeneArt (Thermo Fisher Scientific, Regensburg, Germany). To improve nuclear transport, the synthetic AspBEC10 gene was 5'-extended with a sequence motif encoding the c-myc NLS, (SEQ ID NO: 13) (Dang & Lee. Mol Cell Biol. Vol. 8 No. 10 (1988), 4048 - 4054). Additionally AspBEC10 was fused at the 3'end with a sequence extension encoding one nucleoplasmin NLS (Robbins et al. Cell 64 (1991), 615 - 623), (SEQ ID NO: 14) and two SV40 NLS (SEQ ID NO: 15) (Kalderon et al., Cell 39 (1984), 499-509). The resulting 3861 bps nucleotide full-length synthetic AspBEC10 gene (SEQ ID NO: 16) encodes a protein of 1286 amino acids (SEQ ID NO: 3). Constitutive expression of AspBEC protein was regulated under the control of the strong *Aspergillus nidulans* tef1 promoter (SEQ ID NO: 11) and tef1 terminator (SEQ ID NO: 19). The identity of the assembled synthetic DNA fragments was confirmed by Sanger sequencing.

The final AspBEC10 protein expression cassette was inserted by Gibson Assembly Cloning (NEB, Frankurt, Germany) into an *E. coli* /*A. niger* shuttle vector, containing all necessary genetic elements for episomal propagation and selection of recombinant *E. coli* and *A. niger* cells.

For vector propagation and selection of recombinant *E. coli* cells, the plasmid harbors the pUC derived high-copy ColE1 origin of replication and the pUC derived β-lactamase marker gene (ampR). For autonomous plasmid replication and selection of recombinant A. niger clones, the fungal AMA1 autonomously replicating sequence (Gems et al., Gene 98 (1991), 61 - 67) (SEQ ID NO: 17) and the hygromycin B resistance gene (hph), (Ruiz-Díez B., Journal of Applied Microbiology 2002, 92, 189-195) were cloned into the plasmid. Expression of hph marker gene was under the control of trpC promoter from *Aspergillus. nidulans.*

### Design of the guide RNA (gRNA) expression cassette

The expression of the gRNA, composed of the constant 19 bps BEC family Stem-Loop Sequence (SEQ ID NO: 7) and the *ku70* specific 24 bps target sequence (SEQ ID NO: 18), followed the developed strategy in which the functional gRNA is liberated from a larger transcript by the action of two ribozyme sequences. For this purpose, the specific chimeric gRNA is transcriptionally fused to the hammerhead ribozyme catalytic motif (HH) (SEQ ID NO: 10) at its 5'-end and to the hepatitis delta virus catalytic motif (HDV) (SEQ ID NO: 9) at its 3'-end. Provision of the transcript was driven by the strong constitutive RNA Polymerase II gpdA promoter (PgpdA) (SEQ ID NO: 20) and the trpC terminator (TtrpC) (SEQ ID NO: 12) from *Aspergillus nidulans.*

The complete gRNA expression cassette composed of the gpdA RNA Polymerase II promoter, the designed gRNA flanked by HH and HDV ribozyme sequences and the trpC terminator was provided as synthetic gene fragments by GeneArt (Thermo Fisher Scientific, Regensburg, Germany) and Genewiz (Brooks Life Sciences, Leipzig, Germany).

Finally, the construction of the all-in-one CRISPR/AspBEC10 vector system pTTL70.2-AspBEC10-Sapl-V5 was completed by cloning the synthetic RNA expression cassette in the prepared *E. coli*/*A. niger* shuttle vector, containing the AspBEC10 DNA nuclease expression cassette, by Gibson Assembly Cloning (NEB, Frankfurt, Germany).

The identity of all cloned genetic parts was confirmed by Sanger-Sequencing.

### Example 2: Demonstration of colony reduction and genome editing activity of the BEC10 protein using the newly developed gRNA expression cassette.

Classical CRISPR nucleases (e.g. Cas9 & Cpf1) have the intrinsic ability to process their own gRNAs. Therefore, the expression of a functional gRNAs only requires a species specific RNA Pol III promoter and a sufficient terminator. Surprisingly, similar strategies did not show sufficient genome editing activity when using the newly discovered BEC family nucleases in filamentous fungi (e.g. *Aspergillus niger*). One of the possible reasons for this reduced functionality could be the lacking ability of the BEC family nucleases to efficiently process their gRNAs. To overcome this possible limitation, we developed a strategy using a RNA Pol II promoter and self-splicing elements to preprocess the gRNA in a correct manner.

In example 2.1 and 2.2 a gRNA expression cassette composed of the gpdA RNA Polymerase II promoter, the designed gRNA flanked by HH and HDV ribozyme sequences and the trpC terminator was used in *Aspergillus niger* to demonstrate the genome editing activity of BEC family nucleases in filamentous fungi using a preprocessed gRNA.

### 2.1. Demonstration of the locus specific genome targeting activity

To demonstrate the activity of our newly developed all-in-one vector system a 24 bps spacer sequence (SEQ ID NO: 19) downstream to the nuclease BEC family specific PAM motif 5'-TTN-3' was identified in the *ku70* sequence and inserted into our CRISPR/AspBEC10 all-in-one vector system and the vector system was introduced by protoplast mediated transformation into the NHEJ deficient *ku70⁻* mutant of *Aspergillus niger* WBT-AN-011 (CBS 141599).

In parallel, negative control experiments using the CRISPR/AspBEC10 all-in-one vector system lacking a spacer sequence targeting the *ku70* gene were performed to demonstrate the dependency of the Cas protein to be guided to the target DNA region by a specific spacer.

After transformation and 13 days incubation at 30°C the culture plates were analyzed by counting the number of grown colonies.

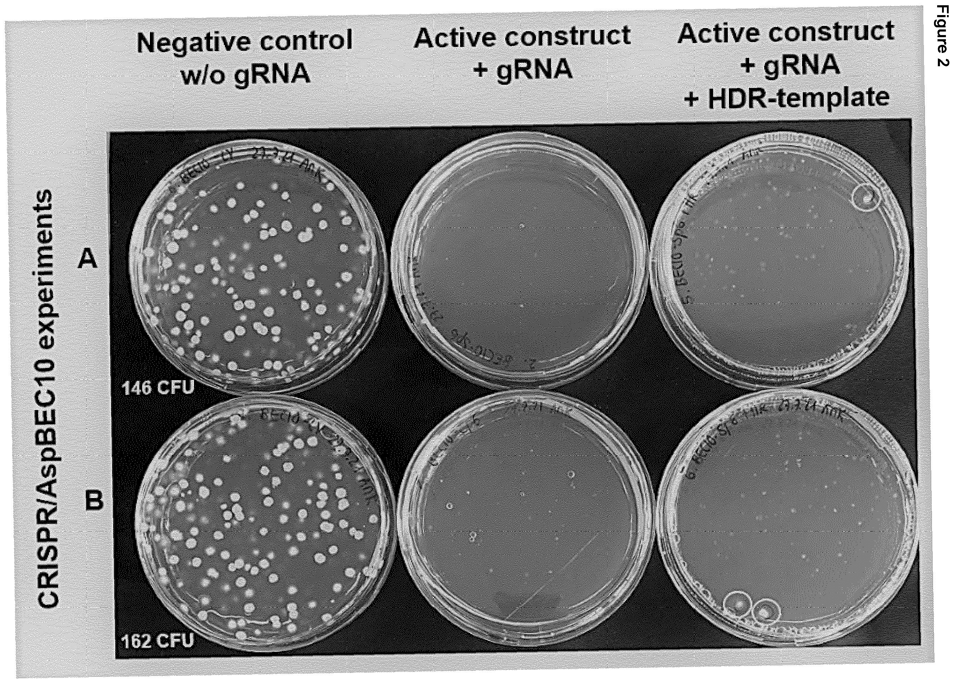

All experiments were carried out in two biological replicates and exemplary plates are shown in Figure 2.

In summary, cells transformed with the negative control constructs (CRISPR/AspBEC10 (without the *ku70* spacer sequence) showed 308 colonies (sum of the grown colonies in both biological replicates). In contrast to this, the active construct (CRISPR/AspBEC10 (with the *ku70* spacer sequence) showed a colony reduction of 100% (0 grown colonies) in comparison to the negative control, proofing the target specific and highly efficient cell depletion induced by the BEC family nuclease.

### 2.2. Demonstration of the editing activity and efficiency

To demonstrate the genome editing activity and efficiency experiments were carried out like described in example 2.1. In addition to the described setup, a 2233 bp homology directed repair template (HDR template) (SEQ ID NO: 21) was co-transfected into the cells. The HDR-template was designed to create a complete chromosomal *ku70⁻* gene knockout by homologous recombination and contains a green fluorescent protein (*gfp*) - expression cassette flanked by homology arms of 500 bps upstream and downstream of the *ku70⁻* target gene. Successful recombination, mediated by the designed HDR-template, will result in replacement of the chromosomal *ku70⁻* gene copy with the gfp-expression cassette and will consequently prevent the programmed CRISPR/AspBEC10 system from targeting the associated PAM and protospacer region.

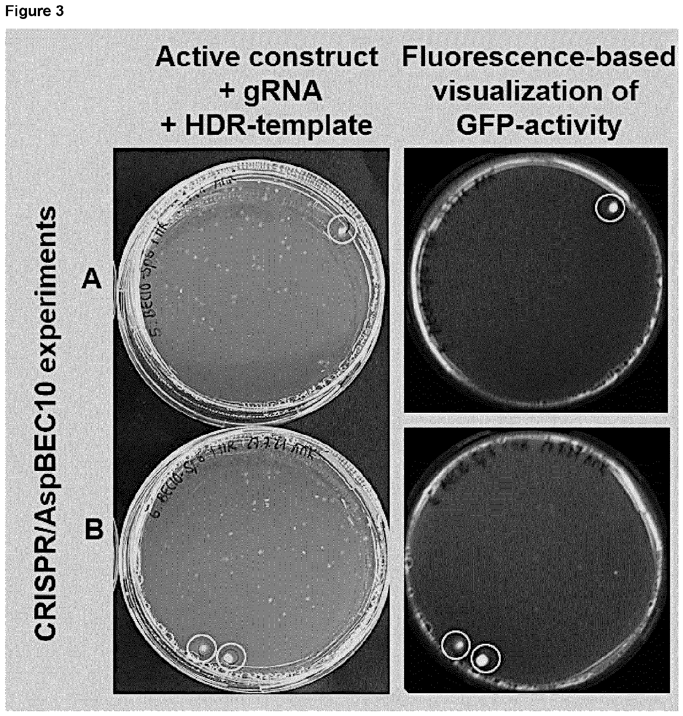

As shown in Figure 3, protoplast-mediated co-transformation of NHEJ deficient strain *Aspergillus niger* WBT-AN-011 (CBS 141599) with 1.5 µg of the programmed CRISPR/AspBEC10 plasmid and 1.5 µg of the HDR-template resulted in selection of 3 recombinant clones of large size that gave fluorescent signals stemming from functional GFP integrated into the *ku70* locus of the *Aspergillus niger* strain proofing the highly efficient genome editing activity induced by the newly developed CRISPR/AspBEC10 system.

In summary, the results generated in Example 2 show the high activity and genome editing efficiency of our newly developed CRISPR/AspBEC10 vector system in *Aspergillus niger.*

### References:

- Arentshorst M, Ram AFJ, Meyer V (2012) Using non-homologous end-joining-deficient strains for functional gene analyses in filamentous fungi. Methods Mol Biol 835: 133 - 150. doi: 10.1007/978-1-61779-501-5_9
- Dang CV, Lee WM. Identification of the human c-myc protein nuclear translocation signal. Mol Cell Biol. 1988; 8:4048-4054
- Robbins J, Dilworth SM, Laskey RA, Dingwall C. Two interdependent basic domains in nucleoplasmin nuclear targeting sequence: identification of a class of bipartite nuclear targeting sequence. Cell. 1991; 64:615-623. doi: 10.1016/0092-8674(91)90245-T
- Nødvig CS, Nielsen JB, Kogle ME, Mortensen UH (2015) A CRISPR-Cas9 System for Genetic Engineering of Filamentous Fungi. PLoSONE 10(7): e0133085. doi:10.1371/journal.pone.0133085
- Gems D, Johnstone IL, Clutterbuck AJ. An autonomously replicating plasmid transforms Aspergillus nidulans at high frequency. Gene. 1991; 98: 61-67.
- Ruiz-Díez B. A Review: Strategies for the transformation of filamentous fungi. Journal of Applied Micro-biology. 2002. pp. 189-195. doi:10.1046/j.1365-2672.2002.01516. xPMID:11849345
- Vanegas, K.G., Jarczynska, Z.D., Strucko, T. et al. Cpf1 enables fast and efficient genome editing in Aspergilli. Fungal Biol Biotechnol 6, 6 (2019)
- Zheng, X., Zheng, P., Sun, J. et al. Heterologous and endogenous U6 snRNA promoters enable CRISPR/Cas9 mediated genome editing in Aspergillus niger. Fungal Biol Biotechnol 5, 2 (2018). https://doi.org/10.1186/s40694-018-0047-4

## Claims

1. A method for modifying a nucleotide sequence at a target site in the genome of the cell of a filamentous fungus comprising introducing into said cell one or more nucleic molecules comprising a first and a second expression cassette,
wherein the first expression cassette comprises a nucleic acid molecule encoding a class 2, type V RNA-guided DNA endonuclease;
and
wherein the second expression cassette comprises a nucleic acid molecule encoding a DNA-targeting and self-splicing RNA comprising from the 5'-end to the 3'-end
(a) a first self-splicing sequence,
(b) a stem loop sequence of SEQ ID NO: 7 or 8 or a sequence being at least 75% identical thereto,
(c) a nucleotide sequence that is complementary to a sequence at the target site; and
(d) a second self-splicing sequence.

2. The method of claim 1, wherein nucleic acid molecule encoding a class 2, type V RNA-guided DNA endonuclease is
(a) a nucleic acid molecule encoding the amino acid sequence of SEQ ID NO: 1, 2 or 3;
(b) a nucleic acid molecule comprising or consisting of the nucleotide sequence of SEQ ID NO: 4, 5, 6 or 16;
(c) a nucleic acid molecule encoding a RNA-guided DNA endonuclease the amino acid sequence of which is at least 80 % identical to the amino acid sequence of (a);
(d) a nucleic acid molecule comprising or consisting of a nucleotide sequence which is at least 80 % identical to the nucleotide sequence of (b); or
(e) a nucleic acid molecule which is degenerate with respect to the nucleic acid molecule of (d).

3. The method of claim 1, wherein the class 2, type V RNA-guided DNA endonuclease is selected from Cpf1 (Cas12a), C2c1 (Cas12b), Cas12c, Cas12d, Cpf1 (Cas12a), Cms1 (Cas12e), Cas12g, Cas12h, Cas12i, Cas12k, Cas12j and Cas14.

4. Use of one or more nucleic molecules comprising a first and a second expression cassette as defined in any one of claims 1 to 3 for modifying a nucleotide sequence at a target site in the genome of a cell of a filamentous fungus.

5. The method of any one of claims 1 to 3 or the use of claim 4, wherein the expression of the second expression cassette is under the control of a constitutive or an inducible promoter, preferably a RNA polymerase II promoter and most preferably the gpdA RNA polymerase II promoter.

6. The method or the use of any preceding claim, wherein the expression from the second expression cassette is under the control of a terminator, preferably a trpC terminator (TtrpC)

7. The method or the use of any preceding claim, wherein the filamentous fungus is selected from *Aspergillus spp*., *Trichoderma spp*, *Penicillium spp*., *Fusarium* spp., *Claviceps* spp., *Hypomyces* spp., *Paecilomyces* spp., *Talaromyces* spp. and *Neurospora spp*., wherein the *Aspergillus spp.* is preferably selected from *Aspergillus niger, Aspergillus nidulans*, *Aspergillus fumigatus*, *Aspergillus oryzae*, *Aspergillus flavus* and *Aspergillus terreus* and is preferably *Aspergillus niger.*

8. The method or the use of any preceding claim, wherein the nucleic acid molecule encoding the RNA-guided DNA endonuclease is codon-optimized for the expression in a filamentous fungus, preferably *Aspergillus niger.*

9. The method or the use of any preceding claim, wherein in the first expression cassette the nucleic acid molecule encoding a RNA-guided DNA endonuclease is extended at the 5'-end and/or the 3'-end by a nuclear localisation signal (NLS), wherein the 5'-NLS is preferably the c-myc NLS and/or the 3'-NLS is preferably the nucleoplasmin NLS and/or one or two SV40 NLS.

10. The method or the use of any preceding claim, wherein the expression from the first expression cassette is under the control of a constitutive or inducible promoter, preferably an *Aspergillus ssp.* tef1 promoter and most preferably an *Aspergillus nidulans* tef1 promoter.

11. The method or the use of any preceding claim, wherein the one or more nucleic molecules are one or two expression vectors and preferably one expression vector.

12. The method or the use of claim 11, wherein the expression vector(s) is/are *E. coli*/*A. niger* shuttle vector(s), wherein the shuttle vector(s) is/are preferably capable of episomal propagation in *E. coli* and *A. niger* cells and/or preferably comprise(s) one or more markers for the selection of recombinant *E. coli* and/or *A. niger* cells.

13. The method or the use of any preceding claim, wherein the first and/or the second self-splicing sequence is/are selected from a hepatitis delta virus (HDV) ribozyme, hammerhead (HH) ribozyme, hairpin (HP) ribozyme, glucosamine-6-phosphate riboswitch (glmS) ribozyme, type-P1 twister ribozyme from rice, varkud satellite (VS) ribozyme, and tRNA self-splicing elements and are preferably selected from a hepatitis delta virus (HDV) ribozyme and a hammerhead (HH) ribozyme.

14. One or more nucleic acid molecules and preferably one nucleic acid molecule comprising a first and a second expression cassette
wherein the first expression cassette comprises a nucleic acid molecule encoding a class 2, type V RNA-guided DNA endonuclease as defined in any one of clams 1 to 3;
and
wherein the second expression cassette comprises a nucleic acid molecule encoding a DNA-targeting and self-splicing RNA comprising from the 5'-end to the 3'-end
(a) a first self-splicing sequence,
(b) a stem loop sequence of SEQ ID NO: 7 or 8 or a sequence being at least 75% identical thereto,
(c) a cloning site into which a nucleotide sequence that is complementary to a sequence at the target site can be added; and
(d) a second self-splicing sequence.

15. A kit for modifying a nucleotide sequence at a target site in the genome of a cell, preferably of a cell of a filamentous fungus,
wherein the kits comprises one or more nucleic molecules comprising a first and a second expression cassette
wherein the first expression cassette comprises a nucleic acid molecule encoding a class 2, type V RNA-guided DNA endonuclease as defined in any one of claims 1 to 3;
and
wherein the second expression cassette comprises a nucleic acid molecule encoding a DNA-targeting and self-splicing RNA comprising from the 5'-end to the 3'-end
(a) a first self-splicing sequence,
(b) a stem loop sequence of SEQ ID NO: 7 or 8 or a sequence being at least 75% identical thereto,
(c) a cloning site into which a nucleotide sequence that is complementary to a sequence at the target site can be added; and
(d) a second self-splicing sequence,
wherein the kit optionally further comprises one or more of
(i) cells, preferably cells of a filamentous fungus,
(ii) a medium for culturing cells and/or the expression of the RNA-guided DNA endonuclease and the DNA-targeting and self-splicing RNA, and
(iii) instructions for using the kit for modifying a nucleotide sequence at a target site in the genome of a cell, preferably of a cell of a filamentous fungus.
